(19) Europäisches Patentamt
European Patent Office
Office européen des brevets

(11) Publication number: 0 169 602
B1

(12) EUROPEAN PATENT SPECIFICATION

(45) Date of publication of patent specification: 01.03.89

(51) Int. Cl.$^4$: C 07 D 205/04

(21) Application number: 85201101.4

(22) Date of filing: 05.07.85

(54) **Preparation of n-substituted azetidine 3-carboxylic acid derivatives.**

(30) Priority: 26.07.84 GB 8419084

(43) Date of publication of application:
29.01.86 Bulletin 86/05

(45) Publication of the grant of the patent:
01.03.89 Bulletin 89/09

(84) Designated Contracting States:
AT BE CH DE FR GB IT LI LU NL SE

(56) References cited:
EP-A-0 001 643
EP-A-0 029 265
FR-A-2 445 828

(73) Proprietor: SHELL INTERNATIONALE
RESEARCH MAATSCHAPPIJ B.V.
Carel van Bylandtlaan 30
NL-2596 HR Den Haag (NL)

(72) Inventor: Mason, Ronald Frank
"Kingswood"
Westwell Ashford-Kent (GB)
Inventor: Briner, Paul Howard
4 Meadowbank Cottages
Painters Forstal Faversham-Kent (GB)

(74) Representative: Hunter, Keith Roger Ian et al
4 York Road
London SE1 7NA (GB)

EP 0 169 602 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the preparation of N-substituted azetidine 3-carboxylic acid derivatives, which are intermediates for the preparation of biologically active compounds, and a novel intermediate in said process.

The production of azetidine derivatives often requires the presence of a 'protective' substituent group on the ring nitrogen atom, which is then removed in order to provide the desired final product, and the benzhydryl group is normally used for this purpose. Thus, for example, European Patent Application No. 0001643 of Shionogi describes a series of azetidine reactions in which the azetidine nitrogen is protected by the benzhydryl(diphenylmethyl) group, including the conversion of the 3-cyano derivative to the corresponding 3-methoxycarbonyl derivative.

However, the introduction of a benzhydryl group is very inconvenient for an economically practicable synthesis route, since the size of that group greatly increases the bulk of material to be processed, only to be removed once its protective function is no longer required. It would be economically very desirable to use a protective group less bulky than the benzhydryl group, and the applicants have developed routes to 3-hydroxyl and 3-cyano azetidines using a benzyl protective group. These routes are described and claimed in, respectively, their UK patent application publication number 2159132 and European published application No. 169 603. When preparing a 3-carboxyl azetidine from the corresponding 3-cyano derivative, the use of a benzyl protective group introduces practical difficulties of compound separation/extraction as a consequence of the high water solubility of N-benzylazetidine-3-carboxylic acid; these problems do not occur when using the benzhydryl analogue, since that carboxylic acid derivative is essentially water insoluble and therefore readily freed from inorganic contaminants/by-products. Applicants have therefore sought a practically convenient route for hydrolysing a N-benzyl-3-cyano azetidine to the corresponding 3-carboxyl derivative, and have surprisingly discovered that conversion via the novel intermediate methyl ester affords a unique combination of process advantages, namely effective separation of the organic ester product from an aqueous reaction medium and the inorganic impurities contained therein, and also the ability of the methyl ester to undergo hydrolysis with hot water.

Accordingly, the present invention provides as a novel intermediate, N-benzyl azetidine-3-carboxylic acid, methyl ester, having the formula I:—

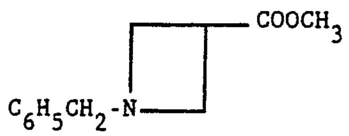

I

together with the use of said methyl ester as an intermediate in the preparation of N-benzyl aetidine-3-carboxylic acid and thence azetidine-3-carboxylic acid. The invention also provides a process for the preparation of N-benzyl azetidine-3-carboxylic acid having the formula II.

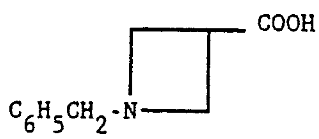

II

which comprises forming N-benzyl azetidine-3-carboxylic acid methyl ester by reacting N-benzyl-3-cyano azetidine with methanol at elevated temperature in the presence of a strong acid, adding water to the reaction mixture, basifying, and separating said methyl ester from the aqueous mixture with a water immiscible organic solvent, removing the organic solvent and hydrolysing said methyl ester with water at elevated temperature.

The reaction of the cyano compound with methanol is effected in the presence of a strong acid, which is suitably an inorganic acid such as hydrogen chloride or sulphuric, with concentrated sulphuric acid usually being preferred, and at elevated temperature which is preferably at least 50°C, suitably as high as 90—100°C, and conveniently under reflux. The methanol and strong acid are desirably utilised in substantially equal volumes; lower amounts of acid adversely affect the reaction rate, while lower amounts of methanol lead to less efficient reflux control and an inconveniently viscous reaction mixture.

After reaction with methanol, the reaction mixture is diluted with water, conveniently combined with cooling by pouring the mixture into a relatively large volume of ice or ice/water. This aqueous mixture is then basified, suitably by the addition of ammonia, and the methyl ester intermediate product is extracted with a water-immiscible organic solvent. Any such solvent having the necessary physical properties and chemical inertness may be used, methylene chloride or paraffinic hydrocarbons, suitably boiling between 60°—80°C, having been found convenient in practice, and the solvent is then removed, suitably by evaporation.

The N-benzyl azetidine-3-carboxylic acid methyl ester intermediate product is then hydrolysed by treatment with hot, preferably boiling, water. The completion of hydrolysis is usually marked by the transformation of the initial oily, two phase system into a homogeneous system, and normally requires about 1 hour. The water may be removed from the final mixture by evaporation, and the desired product, N-benzyl azetidine-3-carboxylic acid, readily isolated as a solid. Alternatively if it is desired to proceed with the ultimate preparation of the deprotected azetidine-3-carboxylic acid, the major part of the water may be removed (e.g. by evaporation), and the crude, wet N-benzyl acid progressed directly to a conventional deprotection treatment, e.g. palladium catalysed hydrogenolysis.

The starting N-benzyl-3-cyano azetidine may be prepared by appropriate adaptations of known synthetic procedures. A convenient synthesis route to that compound is desicribed and claimed in the applicants' European Patent Application Publication No. 169603, namely by reacting the corresponding 3-hydroxyl azetidine with an alkylsulphonyl halide, e.g. mesyl chloride, followed by treatment with an alkali metal cyanide in the presence of a lipophilic phase transfer catalyst such as "Adogen" 464. ("Adogen" is a registered Trade Mark.)

As mentioned above, the N-benzyl-azetidine-3-carboxylic acid, methyl ester of formula I, and the corresponding free acid of formula II, are useful intermediates. Thus, they may be converted by known procedures, for example by hydrogenolysis, to azetidine-3-carboxylic acid derivatives, which exhibit plant growth regulant properties, especially the property of rendering sterile the male parts of plants.

The invention therefore includes the use of N-benzyl-azetidin-3-carboxylic acid methyl ester prepared by the process of the invention, as intermediate for the preparation of N-benzyl azetidine-3-carboxylic acid and azetidine-3-carboxylic acid.

The invention is illustrated in the following Examples.

## Example 1

A) a 250 ml multi-necked flask fitted with Teflon bladed stirrer was charged with N-benzyl-3-cyano-azetidine (17 g) and methanol (60 ml). Concentrated sulphuric acid (60 ml SG 1.84) was added from a dropping funnel over a period of 45 minutes. The reaction was exothermic and the temperature was held in the range 50—55° maximum. After 2 hours at 80° the conversion of starting cyano-azetidine was complete, as determined by GLC analysis. The reaction mixture was drowned in ice (400 g), basified with ammonia (120 ml, .880) and extracted with methylene chloride (2 × 50 ml). The combined extracts were washed with water (50 ml) and stripped on a rotary evaporator. Crude product (17.6 g) was distilled (5 cm. vigreaux) from an oil bath to give N-benzyl azetidine-3-carboxylic acid, methyl ester (15.7 g), b.p. 92—95° at 0.2 mm Hg.

Elemental Analysis:—

| | | | |
|---|---|---|---|
| Found | C = 70.5; | H = 7.7; | N = 6.9% |
| Calc. for $C_{12}H_{15}NO_2$ | C = 70.2; | H = 7.4; | N = 6.8% |

The structure was further confirmed by 360 MHz 'H NMR.

B) The N-benzyl-azetidine-3-carboxylic acid, methyl ester obtained in A) (1.5 g) was mixed with distilled water (25 ml) and a few boiling chips and boiled under a nitrogen blanket. After 40 minutes reflux the initial oily two phase system became homogenous. The water was stripped on a rotary evaporator and the product readily crystallised. After drying in a vacuum oven at 60° the desired product (N-benzyl-azetidine-3-carboxylic acid) (1.4 g) m.p. 152—154° was obtained.

Elemental Analysis:—

| | | | |
|---|---|---|---|
| Found | C = 68.7;% | H = 7.0; | N = 7.2% |
| Calc. for $C_{12}H_{15}NO_2$ | C = 69.1; | H = 6.9; | N = 7.3 |

The structure was further confirmed by 360 MHz 'H NMR.

## Example 2

The procedure of Example 1A) was repeated, except that the post-basifying extraction was carried out with a commercial mixture of $C_5$ to $C_8$ paraffinic hydrocarbons boiling between 60° and 80°C. This produced the desired methyl ester in a yield of 82%. Wiped film distillation (168°C, 5 mm Hg) yielded this product in 91% purity.

## Example 3

Use of N-benzyl-azetidine-3-carboxylic acid to prepare azetidin-3-carboxylic acid

1-Benzylazetidine-3-carboxylic acid (0.5 g, prepared as in Example 1) in methanol (15 ml) was hydrogenated in the presence of a 5% palladium on carbon catalyst at room temperature. The catalyst was filtered off and the solvent removed from the filtrate under reduced pressure to give azetidin-3-carboxylic acid in 90% yield.

Example 4
(Comparative)

A) The ethyl and isopropyl, esters of N-benzyl azetidine-3-carboxylic acid were prepared following procedures similar to that described in Example 1, but replacing the methanol with, respectively, ethanol or isopropanol. In the latter case, the yield was low when using concentrated sulphuric acid as the strong acid, but was increased to 70% by replacing that acid with gaseous hydrogen chloride. The structure of each ester was confirmed by NMR, 1R and Mass Spec.; the elemental analyses were as follows:—

| | | | |
|---|---|---|---|
| Ethyl Ester. Found | C = 71.2; | H = 7.9; | N = 6. 3% |
| Calc. for $C_{13}H_{17}NO_2$ | C = 71.2; | H = 7.8; | N = 6.4% |
| Isopropyl Ester. Found | C = 72.2; | H = 8.6; | N = 6.3% |
| Cal. for $C_{14}H_{19}NO_2$ | C = 72.1; | H = 8.2; | N = 6.0% |

B) Each of the esters prepared in Example 3A) was hydrolysed by refluxing 1 g in 10 ml of distilled water; the hydrolysis proceeded quite differently from that of the methyl ester. That methyl ester gave a clear solution (the criterion for complete conversion) in 37—40 minutes, whilst the ethyl ester requires 6 hours and the isopropyl ester 24—40 hours. Thus, the hydrolysis time (using boiling water) for the ethyl and isopropyl esters is inconveniently long for a commercial scale of synthesis, whereas the methyl ester hydrolyses very rapidly under those same, convenient conditions.

## Claims

1. N-benzyl azetidine-3-carboxylic acid methyl ester, having the formula I:—

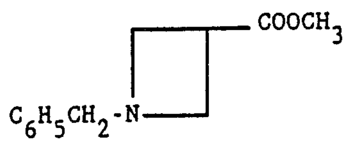

I

2. Process for the preparation of N-benzyl azetidine-3-carboxylic acid or azetidine-3-carboxylic acid comprising hydrolysing N-benzyl azetidine-3-carboxylic acid methyl ester with water at elevated temperature to yield N-benzyl azetidine-3-carboxylic acid optionally followed by removal of the N-benzyl protecting group to give azetidine-3-carboxylic acid.

3. Process for the preparation of N-benzyl azetidine 3-carboxylic acid having the formula II:—

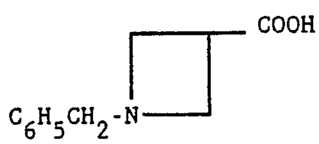

II

which comprises forming the azetidine carboxylic acid methyl ester as defined in claim 1 by reacting N-benzyl-3-cyano azetidine with methanol at elevated temperature in the presence of a strong acid, adding water to the reaction mixture, basifying, and separating said methyl ester from the aqueous mixture with a water-immiscible organic solvent, removing the organic solvent and hydrolysing said methyl ester with water at elevated temperature.

4. Process as claimed in claim 3 wherein the strong acid is hydrogen chloride or sulphuric acid.

5. Process as claimed in claim 3 or 4 wherein the methanol and strong acid are utilised in substantially equal volumes.

6. Process as claimed in claims 3, 4 or 5 wherein the reaction of the 3-cyano azetidine starting material with methanol is effected at a temperature of at least 50°C.

7. Process as claimed in any one of claims 3—6, wherein the product from the methanol reaction is added to a relatively large volume of ice, and basified by treatment with ammonia.

8. Process as claimed in any one of claims 3—7, wherein the methyl ester is extracted from the aqueous mixture with methylene chloride, or a paraffinic hydrocarbon.

9. Process as claimed in any one of claims 3—8 wherein the hydrolysis of the methyl ester is effected by the addition of water and boiling the resulting aqueous mixture.

**Revendications**

1. Ester méthylique d'acide N-benzylazétidine-3-carboxylique, répondant à la formule I:

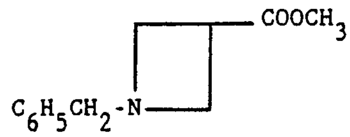

I

2. Procédé de préparation d'acide N-benzylazétidine-3-carboxylique ou d'acide azétidine-3-carboxylique, comprenant l'hydrolyse de l'ester méthylique d'acide N-benzylazétidine-3-carboxylique avec de l'eau à une température élevée, pour donner l'acide N-benzylazétidine-3-carboxylique, éventuellement suivie de l'élimination du groupe protecteur N-benzyle pour donner l'acide azétidine-3-carboxylique.

3. Procédé de préparation de d'acide N-benzylazétidine-3-carboxylique répondant à la formule II:

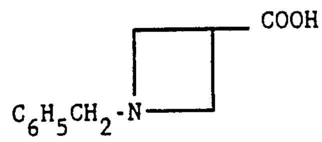

II

qui comprend les étapes qui consistent à former l'ester méthylique d'acide N-benzylazétidine-3-carboxylique selon la revendication 1, en faisant réagir la N-benzyl-3-cyanoazétidine avec du méthanol à une température élevée, en présence d'un acide fort, à ajouter de l'eau au mélange réactionnel, à l'alcaliniser et à séparer ledit ester méthylique d'avec le mélange aqueux à l'aide d'un solvant organique non miscible à l'eau, à éliminer le solvant organique et à hydrolyser ledit ester méthylique avec de l'eau à une température élevée.

4. Procédé selon la revendication 3, dans lequel l'acide fort est le chlorure d'hydrogène ou l'acide sulfurique.

5. Procédé selon la revendication 3 ou 4, dans lequel le méthanol et l'acide fort sont utilisés en volumes essentiellement égaux.

6. Procédé selon la revendication 3, 4 ou 5, dans lequel la réaction de la substance de départ 3-cyanoazétidine avec le méthanol est réalisée à une température d'au moins 50°C.

7. Procédé selon l'une quelconque des revendications 3 à 6, dans lequel le produit de la réaction avec le méthanol est ajouté a un volume relativement important de glace, et alcalinisé par traitement avec de l'ammoniaque.

8. Procédé selon l'une quelconque des revendications 3 à 7, dans lequel l'ester méthylique est extrait du mélange aqueux avec du chlorure de méthylène ou avec un hydrocarbure paraffinique.

9. Procédé selon l'une quelconque des revendications 3 à 8, dans lequel on effectue l'hydrolyse de l'ester méthylique en ajoutant de l'eau et en portant le mélange aqueux résultant à ébullition.

**Patentansprüche**

1. N-Benzyl-azetidin-3-carbonsäure-methylester mit der Formel I:

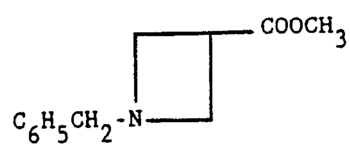

I

2. Verfahren zur Herstellung von N-Benzyl-azetidin-3-carbonsäure oder von Azetidin-3-carbonsäure, umfassend ein Hydrolysieren von N-Benzyl-azetidin-3-carbonsäure-methylester mit Wasser bei erhöhter Temperatur zur Ausbildung von N-Benzyl-azetidin-3-carbonsäure, gewünschtenfalls mit anschließender Abtrennung der N-Benzylschutzgruppe zur Ausbildung von Azetidin-3-carbonsäure.

3. Verfahren zur Herstellung von N-Benzyl-azetidin-3-carbonsäure mit der Formel II:

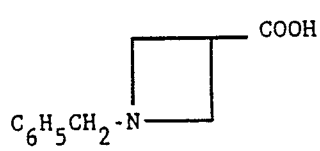

II

welches Verfahren ein Ausbilden des Azetidin-carbonsäure-methyl-esters, wie in Anspruch 1 definiert, durch Umsetzen von N-Benzyl-3-cyano-azetidin mit Methanol bei erhöhter Temperatur in Anwesenheit einer starken Säure, ein Zusetzen von Wasser zum Reaktionsgemisch, ein Basischstellen, und ein Abtrennen des Methylesters aus dem wäßrigen Gemisch mit einem mit Wasser nicht mischbaren organischen Lösungsmittel, ein Abtrennen des organischen Lösungsmittels und ein Hydrolysieren des Methylesters mit Wasser bei erhöhter Temperatur umfaßt.

4. Verfahren nach Anspruch 3, worin die starke Säure Chlorwasserstoff oder Schwefelsäure ist.

5. Verfahren nach Anspruch 3 oder 4, worin das Methanol und die starke Säure in im wesentlichen gleichen Volumina verwendet werden.

6. Verfahren nach Anspruch 3, 4 oder 5, worin die Umsetzung des 3-Cyano-azetidin-Ausgangsmaterials mit Methanol bei einer Temperatur von wenigstens 50°C ausgeführt wird.

7. Verfahren nach einem der Ansprüche 3 bis 6, worin das Produkt aus der Methanolumsetzung zu einem verhältnismäßig großen Volumen Eis zugesetzt und durch Behandlung mit Ammoniak basisch gemacht wird.

8. Verfahren nach einem der Ansprüche 3 bis 7, worin der Methylester mit Methylenchlorid oder einem Paraffinkohlenwasserstoff aus dem wäßrigen Gemisch extrahiert wird.

9. Verfahren nach einem der Ansprüche 3 bis 8, worin die Hydrolyse des Methylesters durch Zubage von Wasser und Kochen des gebildeten wäßrigen Gemisches ausgeführt wird.